## Europäisches Patentamt
## European Patent Office
## Office européen des brevets

(19)

(11) Veröffentlichungsnummer: **0 030 718**
**B1**

(12)

# EUROPÄISCHE PATENTSCHRIFT

(45) Veröffentlichungstag der Patentschrift:
**01.12.82**

(51) Int. Cl.³: **C 12 Q 1/48,** C 12 Q 1/44,
C 12 Q 1/00

(21) Anmeldenummer: **80107835.3**

(22) Anmeldetag: **11.12.80**

(54) Verfahren und Reagens zur Bestimmung von Triglyceriden.

(30) Priorität: **14.12.79 DE 2950381**

(43) Veröffentlichungstag der Anmeldung:
**24.06.81 Patentblatt 81/25**

(45) Bekanntmachung des Hinweises auf die Patenterteilung:
**01.12.82 Patentblatt 82/48**

(84) Benannte Vertragsstaaten:
**AT BE CH DE FR GB IT LI LU NL SE**

(56) Entgegenhaltungen:
**DE-A-1 961 983**
**DE-A-2 535 953**
**DE-A-2 737 286**
**DE-A-2 738 184**

**CHEMICAL ABSTRACTS, Band 83, Nr. 15, 13. Oktober 1975, Seite 372, Zusammenfassung Nr. 129984s, Columbus, Ohio, US, A. ATKINSON et al.: "Behavior of bacillus stearothermophilus grown in different media"**

**CHEMICAL ABSTRACTS, Band 74, Nr. 7, 15. Februar 1971, Seite 233, Zusammenfassung Nr. 34968q, Columbus, Ohio, US, G. BENZONANA et al.: "Interactions between bile acids and fatty acids. Behavior of mixed solutions at air-water and oil-water interfaces"**

(73) Patentinhaber: **BOEHRINGER MANNHEIM GMBH, Sandhofer Strasse 112-132 Postfach 31 01 20, D-6800 Mannheim 31-Waldhof (DE)**

(72) Erfinder: **Klose, Sigmar, Dr., Breitenloh 7, D-8131 Berg 2 (DE)**
Erfinder: **Röder, Albert, Dr., Bahnhofstrasse 41, D-8124 Seeshaupt (DE)**
Erfinder: **Schneider, Walter, Geistbühelstrasse 27, D-8120 Weilheim (DE)**

(74) Vertreter: **Weickmann, Heinrich, Dipl.-Ing. et al, Patentanwälte Dipl.-Ing. H. Weickmann Dipl.-Phys.Dr. K. Fincke Dipl.-Ing. F.A. Weickmann Dipl.-Chem. B. Huber Dr.-Ing. H. Liska Möhlstrasse 22, D-8000 München 86 (DE)**

(56) Entgegenhaltungen:
**CHEMICAL ABSTRACTS, Band 83, Nr. 5, 4. August 1975, Seite 241, Zusammenfassung Nr. 39967j, Columbus, Ohio, US, J.A. BUSWELL et al.: "Utilization of phenol and cresols by Bacillus. stearothermophilus, strain PH24"**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Verfahren und Reagens zur Bestimmung von Triglyceriden

Die Erfindung betrifft ein Verfahren und ein Reagens zur Bestimmung von Triglyceriden durch Esterspaltung mittels Lipase und gegebenenfalls Esterase.

Ein bekanntes Verfahren zur Bestimmung der Triglyceride in biologischem Material, vorzugsweise in Serum, basiert auf der folgenden Reaktionsgleichungsfolge:

1) Triglyceride $\xrightarrow{\text{Lipase/Esterase}}$ Fettsäuren + Glycerin

2) Glycerin + ATP $\xrightarrow{\text{GK}}$ Glycerin-1-phosphat + ADP

GK = Glycerokinase

Bekannt ist weiter, dass für den ersten Reaktionsschritt gemäss Gleichung 1) Aktivatoren zugesetzt werden müssen, die der Lipase bzw. den Lipolyseenzymen (bei vielen Lipasepräparaten lässt sich durch Esterasezusatz die Spaltungsrate verbessern) überhaupt erst eine Spaltung der Triglyceride ermöglichen. Als Aktivatoren werden im allgemeinen oberflächenaktive Mittel verwendet. Jedoch können auch substituierte Arylalkohole, wie z.B. Dichlorphenol, zusammen mit den oberflächenaktiven Verbindungen eingesetzt werden und führen zu einer noch besseren Aktivierung.

Ein wesentlicher Nachteil der Aktivatoren besteht jedoch darin, dass sie die Glycerokinase (EC. 2.7.1.30), welche im zweiten Reaktionsschritt entsprechend Gleichung 2) benötigt wird, nachteilig beeinflussen. Dies wird durch die nachfolgende Tabelle 1 veranschaulicht, welche für eine bekannte Glycerokinase die Stabilitätsdaten in Gegenwart und Abwesenheit von Stabilisatoren zeigt:

Tabelle 1
Stabilität von GK aus Cand. myc. unter Einfluss verschiedener Zusätze; Temperatur 25°C

| Zusatz | Reaktivität in % nach | | | | |
| --- | --- | --- | --- | --- | --- |
| | 0,5 h | 1 h | 2 h | 3 h | 4 h |
| ohne | – | 95 | 92 | 86 | 80 |
| Alkylarylpolyäthylenoxidäther | – | 90 | 76 | 67 | 57 |
| Taurodesoxycholat | – | 78 | 61 | 40 | 23 |
| 3,5-Dichlorphenol | <5 | 0 | 0 | 0 | 0 |

Daher war es bisher nötig, die Glycerokinase erst unmittelbar vor Durchführung der Bestimmung zuzusetzen. Für das Reagens bedeutet dies einen wesentlichen Nachteil. Denn bei der modernen Arbeitstechnik ist es wünschenswert, über Reagenzien zu verfügen, die in einer einzigen gebrauchsfertigen Mischung sämtliche Komponenten mit möglichst grosser Stabilität enthalten. Erst unter dieser Voraussetzung ist ein rationelles Arbeiten insbesondere für Analysenautomaten möglich.

Aufgrund der Instabilität der Glycerokinase in Gegenwart der erwähnten Aktivierungsmittel für die Lipasereaktion wurde bisher so vorgegangen, dass man ein Reagensgemisch ohne Glycerokinase mit der Probe mischte, die Leerwertreaktion ablaufen liess (ca. 10 Minuten), dann nach Ablesen einer ersten Extinktion die Glycerokinase zufügte und nach Ablauf von weiteren ca. 10 Minuten eine zweite Extinktion bestimmte. Aus der Extinktionsdifferenz war dann die Triglyceridkonzentration zu berechnen. Das Reagens enthielt dabei noch ein System zur Bestimmung von gebildetem ADP. Es wäre jedoch auch möglich, statt dessen gebildetes Glycerin-1-phosphat zu messen.

Der Erfindung liegt die Aufgabe zugrunde, bei einem Verfahren und einem Mittel zur Bestimmung der Triglyceride einen Weg zu finden, um Stabilität der Glycerokinase in Gegenwart der erwähnten Aktivierungsmittel so zu verbessern, dass die Notwendigkeit beseitigt wird, dieses Enzym erst unmittelbar vor der Reaktion zuzusetzen.

Nunmehr wurde überraschenderweise gefunden, dass eine Glycerokinase aus Bacillus stearothermophilus in Gegenwart der Aktivatoren beständig ist und daher durch Verwendung dieses Enzyms die erwähnten Nachteile überwunden werden können.

Ein Gegenstand der Erfindung ist daher ein Verfahren zur Bestimmung von Triglyceriden durch Esterspaltung mittels Lipase und Esterase unter Bildung von Fettsäuren und Glycerin, Phosphorylierung des Glycerins mit ATP in Gegenwart von Glycerinkinase unter Bildung von Glycerin-1-phosphat und ADP und Bestimmung einer dieser beiden gebildeten Substanzen, welches dadurch gekennzeichnet ist, dass eine Glycerokinase aus Bacillus stearothermophilus in Kombination mit einem Aktivator aus der Gruppe der Detergentien oder/und Phenolderivate oder/und Anilinderivate eingesetzt wird.

Dass Bacillus stearothermophilus eine Glycerokinase enthält, ist bekannt aus J. Appl. Bac. 38 (1975) 301–304. Es konnte daraus jedoch nicht entnommen werden, dass diese Glycerokinase gegenüber der oben angegebenen Gruppe von Aktivatoren eine gute Stabilität aufweisen würde, die die Verwendung zusammen mit diesen Aktivatoren ermöglicht. Diese überlegene Stabilität gegenüber einigen typischen Aktivatoren im Vergleich zu Glycerokinase aus anderem biologischem Material zeigen die in der nachstehenden Tabelle 2 aufgeführten Aktivitätswerte.

Tabelle 2
Stabilität von Glycerokinase aus verschiedenen
Mikroorganismen, Temperatur 25°C

| GK aus | Konzen-tration Dichlor-phenol | Restaktivität nach | |
|---|---|---|---|
| | | 30 Min. | 60 Min. |
| Candida mycoderma | 2 mM 5 mM | 68% 18% | 56% 7% |
| Bac. stearothermo-philus | 2 mM 5 mM | 100% 100% | 100% 100% |
| E. coli (handelsübl.) | 2 mM 5 mM | 93% 81% | 87% 58% |

Als Aktivator wird im Rahmen der Erfindung vorzugsweise ein Detergenz aus der Gruppe der Polyäthylenoxidester oder -äther, der Gallensäuren oder ein Alkylsulfat allein oder im Gemisch verwendet. Aus der Gruppe der Phenole und Aniline werden solche Aktivatoren bevorzugt, welche mit einer Carboxylgruppe, Sulfonsäuregruppe, einem, zwei oder drei Halogenatomen oder/und einer oder zwei Nitrogruppen substituiert sind. Unter den Halogenatomen werden Chlor und Brom bevorzugt.

Beispiele für zur Gewinnung des erfindungsgemäss verwendeten Enzyms geeignete Stämme der Art Bacillus stearothermophilus sind neben der in der oben erwähnten Literaturstelle J. Appl. Bact. genannten NCIB 8924 (NCA 1503; ATCC 7954) auch die aus der DE-OS 2738184 bekannten Mutanten NCIB 111270 und 111271. Auch andere Stämme von Bacillus stearothermophilus mit einem die Aufarbeitung lohnenden Gehalt an Glycerokinase können verwendet werden. Zweckmässig wird die Züchtung der Biomasse auf einem glycerinhaltigen Medium durchgeführt, da in vielen Fällen hierdurch ein höherer Gehalt an Enzym in der Biomasse erreicht wird.

Wie oben schon erwähnt, wird beim erfindungsgemässen Verfahren in Gleichung 2) gebildetes Glycerin-1-phosphat oder ADP bestimmt, wobei bekannte Methoden zur Anwendung kommen können.

Gemäss einer bevorzugten Ausführungsform setzt man ADP in an sich bekannter Weise durch Phosphorylierung mit Phosphoenolpyruvat in Gegenwart von Pyruvatkinase unter Bildung von ATP und Pyruvat um, oxidiert letzteres mit NADH in Gegenwart von Lactatdehydrogenase (LDH) zu NAD und misst dieses in üblicher Weise.

Alternativ setzt man beim Verfahren der Erfindung Glycerin-1-phosphat in an sich bekannter Weise mit $O_2$ in Gegenwart von Glycerin-1-phosphatoxidase zu Dihydroxyacetonphosphat und $H_2O_2$ um, lässt letzteres mit 4-Aminoantipyrin und einem Phenol- oder Anilinderivat in Gegenwart von Peroxidase zu einem Farbstoff reagieren und misst diesen.

Gemäss einer weiteren Ausführungsform des erfindungsgemässen Verfahrens setzt man ADP in an sich bekannter Weise durch Phosphorylierung mit Phosphoenolpyruvat in Gegenwart von Pyruvatkinase zu ATP und Pyruvat um, lässt gebildetes Pyruvat mit Sauerstoff und Pyruvatoxidase zu Acetylphosphat $CO_2$ und $H_2O_2$ reagieren und bestimmt letzteres wiederum wie vorstehend erwähnt.

Ein weiterer Gegenstand der Erfindung ist ein Reagenz zur Bestimmung von Triglyceriden enthaltend Lipase, Esterase, ATP, Glycerokinase und ein System zur Bestimmung von Glycerin-1-phosphat oder ATP, welches gekennzeichnet ist durch einen Gehalt an Glycerokinase aus Bacillus stearothermophilus in Kombination mit einem Aktivator aus der Gruppe der Detergentien oder/und Phenolderivate oder/und Anilinderivate.

Für die bevorzugten Aktivatoren im erfindungsgemässen Reagens gelten die obigen Ausführungen zum Verfahren in gleicher Weise. So wird ein Gehalt an Polyäthylenoxidester oder -äther, einer Gallensäure oder/und einem Alkylsulfat als Detergens bevorzugt. Als Phenol oder Anilin wird vorzugsweise ein solches verwendet, welches mit einer Carboxygruppe, Sulfonsäuregruppe, einem, zwei oder drei Halogenatomen oder/und einer oder zwei Nitrogruppen substituiert ist. Brom und Chlor werden als Halogen bevorzugt.

Das System zur Bestimmung von ADP besteht bei einer bevorzugten Ausführungsform des Reagens aus Phosphoenolpyruvat, Pyruvatkinase, NADH und Lactatdehydrogenase. Als Aktivator wird vorzugsweise Natriumdodecylphosphat verwendet. Eine andere, in diesem Zusammenhang bevorzugte Aktivatorkombination besteht aus Dichlorphenol, Natriumcholat und einem Alkylarylpolyäthylenoxidäther.

Ein bevorzugtes Reagens dieser Zusammensetzung enthält
60 bis 200 U Lipase,
0,4 bis 10 U Esterase,
0,5 bis 10 U Glycerokinase aus Bacillus stearothermophilus,
0,5 bis 10 U Pyruvatkinase,
2 bis 10 U Lactatdehydrogenase,
0,25 bis 0,7 μMol Phosphoenolpyruvat,
0,25 bis 0,7 μMol ATP,
0,15 bis 0,3 μMol NADH,
0,3 bis 0,5 μMol Natriumdodecylsulfat,
4 bis 100 μMol $MgSO_4$ und
20 bis 100 μMol Phosphatpuffer, pH 6,8 bis 7,5,
bezogen auf 1 ml Reagenslösung.

Ein weiteres bevorzugtes Reagens dieser Art enthält
0,6 bis 10 U Esterase,
60 bis 300 U Lipase,
0,5 bis 10 U Glycerokinase aus Bacillus stearothermophilus,
0,5 bis 10 U Pyruvatkinase,
2 bis 10 U Lactatdehydrogenase,
0,15 bis 3 μMol NADH,

0,25 bis 0,7 μMol Phosphoenolpyruvat,
0,25 bis 0,7 μMol ATP,
1,5 bis 4 μMol 2,3-Dichlorphenol,
1,5 bis 5,0 mg Natriumcholat,
0,8 bis 2 μl Octylphenylpolyäthylenoxidester (9 bis 10 Äthoxyeinheiten),
150 bis 300 μMol $MgSO_4$ und
40 bis 100 μMol Tris/Weinsäurepuffer, pH 7,0 bis 7,6, bezogen auf 1 ml Reagenslösung.

Ein anderes bevorzugtes Reagens der Erfindung enthält als System zur Bestimmung von Glycerin-1-phosphat Glycerinphosphatoxidase, Peroxidase und 4-Aminoantipyrin. Als Aktivator enthält es bevorzugt 2,4-Dichlorphenol zusammen mit einem Cholat und einem Polyäthylenoxidäther, besonders bevorzugt Isotridecylpolyäthylenoxidäther.

Eine besonders bevorzugte Zusammensetzung für ein derartiges Reagens enthält
0,6 bis 10 U Esterase,
60 bis 300 U Lipase,
0,5 bis 10 U Glycerokinase aus Bacillus stearothermophilus,
2 bis 10 U Glycerinphosphatoxidase,
0,3 bis 1 μMol ATP,
3 bis 10 μMol 2,4-Dichlorphenol,
1,5 bis 5,0 mg Natriumcholat,
1,5 bis 10 μl Isotridecylpolyäthylenoxidäther,
150 bis 300 μMol Magnesiumsulfat,
40 bis 100 μMol Puffer, pH 7,5 bis 8,5 bezogen auf 1 ml Reagenslösung.

Das erfindungsgemässe Reagens besteht aus einer Mischung aller angegebenen Komponenten in fester oder gelöster Form. Aufgrund der grossen Stabilität aller seiner Bestandteile eignet es sich auch besonders zur Imprägnierung von festen Trägersubstanzen, wie Papier, Kunststofffolien und dergleichen. Derartige imprägnierte Trägersubstanzen eignen sich besonders für Schnelltests.

Die überlegene Stabilität eines erfindungsgemässen Reagens im Vergleich zu einem gleich zusammengesetzten Reagens mit einer Glycerokinase aus anderem Ausgangsmaterial zeigt nachstehende Tabelle 3.

Tabelle 3
Vergleich der Stabilität von Reagenzien zur Triglyceridbestimmung mit Glycerokinase aus Cand. myc. und Bac. stearothermophilus (erfindungsgemäss)
Reagens: Phosphatpuffer 20 mM, ph = 7; 4 mM $MgSO_4$; 0,35 mM Natriumdodecylsulfat; 0,2 mM NADH; 0,44 mM ATP; 0,36 mM PEP; 6 U/ml LDH; 1 U/ml PK; 80 U/ml Lipase; 0,6 U/ml Esterase

| Standzeit in Stunden | GK (Cand. myc.) Restaktivität in % 4°C | GK (Bac. stearotherm.) Restaktivität in % 4°C |
|---|---|---|
| 0,5 | 5 | 100 |
| 1 | 6 | 100 |
| 5 | 2 | 94 |
| 8 | – | 100 |
| 50 | – | 98 |

Soweit im Verfahren NADH gebildet wird, ist es auch möglich, dieses in Gegenwart eines Elektronenüberträgers, wie Diaphorase, Phenacinmetosulfat oder Meldolablau mit einem Tetrazoliumsalz in ein gefärbtes Formazan zu überführen, welches in der für Farbstoffe üblichen Weise gemessen werden kann.

Die folgenden Beispiele erläutern die Erfindung weiter. Folgende Abkürzungen werden darin verwendet:

ATP = Adenosintriphosphat
ADP = Adenosindiphosphat
PEP = Phosphoenolpyruvat
NADH = Nicotinamid-Adenin-Dinucleotid (reduzierte Form)
NAD = Nicotinamid-Adenin-Dinucleotid (oxidierte Form)
GK = Glycerokinase
PK = Pyruvatkinase
LDH = Lactatdehydrogenase
POX = Pyruvatoxidase

Beispiel 1
Bestimmung von ADP
Reagens 1:
20 mM Phosphatpuffer, pH 7
4 mM $MgSO_4$
0,35 mM Na-Dodecylsulfat
0,2 mM NADH
0,44 mM ATP
0,36 mM PEP
6 U/ml LDH
1 U/ml PK

80 U/ml Lipase
0,6 U/ml Esterase
Reagens 2: identisch mit Reagens 1 + 1 U/ml GK

Testdurchführung:
In zwei optisch abgeglichene Küvetten (1 cm opt. Weglänge) werden 2,5 ml Reagens 1 in Küvette 1 pipettiert bzw. 2,5 ml Reagens 2 in Küvette 2 pipettiert. Dann werden in jede Küvette 0,050 ml Probe pipettiert und 15 Minuten bei Raumtemperatur stehengelassen. Danach wird die Extinktion bei 365 nm abgelesen.
Auswertung: $(E_1-E_2) \times 1318 =$ mg Triglyceride/100 ml

Beispiel 2
Bestimmung von ADP
Reagens 1:
60 mM Tris/Weinsäurepuffer, pH 7,3

3) $\text{Glycerin-1-phosphat} + O_2 \xrightarrow[\text{Dihydroxyaceton} + H_2O_2]{\text{Glycerinphosphat-oxidase}}$

4) $\text{2,4-Dichlorphenol} + \text{4-Aminoantipyrin} + H_2O_2 \xrightarrow{\text{POD}}$
$\text{chinoider Farbstoff} + H_2O$

Reagens:
60 mM Tris/Weinsäurepuffer, pH 8
200 mM MgSO₄
5 mM 2,4-Dichlorphenol
1 ml/l Isotridecyläther
1,7 g/l Na-Cholat
0,4 mM ATP
3 U/ml Glycerinphosphat-oxidase[+)]
1 U/ml GK

[+)] Arch. Biochem. Biophys. 80 (1960) 250–255

1 mM 4-Aminoantipyrin
80 U/ml Lipase
1 U/ml Esterase
Testdurchführung:
2,0 ml Reagens und 0,020 ml Probe werden in eine Küvette pipettiert und 15 Minuten bei Raumtemperatur inkubiert. Dann wird die Extinktion bei 500 nm abgelesen. Die Auswertung erfolgt nach Abzug eines Reagenzienleerwerts vom gemessenen Wert durch Vergleich mit einem Triglycerid-Standard bekannter Konzentration.

Beispiel 4
Bestimmung von ADP gemäss folgenden Reaktionsgleichungen:

5) $\text{ADP} + \text{PEP} \xrightarrow{\text{PK}} \text{ATP} + \text{Pyruvat}$

6) $\text{Pyruvat} + PO_4^{3-} + O_2 \xrightarrow{\text{POX}[+)]} \text{Acetylphosphat} + CO_2 + H_2O$

7) wie Reaktion 4) in Beispiel 3

[+)] POX = Pyruvatoxidase

200 mM MgSO₄
2 mM 2,3-Dichlorphenol
1 ml/l Octylphenylpolyäthylenoxidester
1,0 g/l Na-Cholat
0,44 mM ATP
0,36 mM PEP
0,2 mM NADH
6 U/ml LDH
1 U/ml PK
80 U/ml Lipase
1 U/ml Esterase
Reagens 2: identisch mit Reagens 1 + 1 U/ml GK
Testdurchführung und Auswertung erfolgt wie in Beispiel 1.

Beispiel 3
Bestimmung von Glycerin-1-phosphat nach folgenden Reaktionsgleichungen:

Reagens:
60 mM Tris/Weinsäurepuffer, pH 7,3
4 mM MgSO₄
10 mM 2,4-Dichlorphenol
1 ml/l Isotridecyläther
1,7 g/l Na-Cholat
0,1 mM ATP
0,1 mM PEP
80 U/ml Lipase
1 U/ml Esterase
1 U/ml GK
1 U/ml POX
1 U/ml PK

Testdurchführung:
20 µl Probe und 3 ml Reagens werden 20 Minuten inkubiert; dann wird die Extinktion bei 500 nm gemessen, von dieser wird die Extinktion eines Reagenzienleerwerts abgezogen. Über Eichung mit einem Standard wird aus der Extinktionsdifferenz die Triglycerid-Konzentration in der unbekannten Probe errechnet.

**Patentansprüche**

1. Verfahren zur Bestimmung von Triglyceriden durch Esterspaltung mittels Lipase und gegebenenfalls Esterase unter Bildung von Fettsäuren und Glycerin, Phosphorylierung des Glycerins mit ATP in Gegenwart von Glycerinkinase unter Bildung von Glycerin-1-phosphat und ADP und Bestimmung einer dieser beiden gebildeten Substanzen, dadurch gekennzeichnet, dass eine Glycerokinase aus Bacillus stearothermophilus in Kombination mit einem Aktivator aus der Gruppe der Detergentien oder/und Phenolderivate oder/und Anilinderivate eingesetzt wird.

2. Verfahren nach Anspruch 1, dadurch gekennzeichnet, dass man als Aktivator ein Detergens aus der Gruppe der Polyäthylenoxidester oder -äther, Gallensäuren oder Alkylsulfate verwendet.

3. Verfahren nach Anspruch 1 oder 2, dadurch gekennzeichnet, dass man als Aktivator ein Phenol oder Anilin verwendet, welches mit einer Carboxylgruppe, Sulfonsäuregruppe, einem, zwei oder drei Halogenatomen oder/und einer oder zwei Nitrogruppen substituiert ist.

4. Verfahren nach einem der vorhergehenden Ansprüche, dadurch gekennzeichnet, dass man einen Bacillus stearothermophilus NCIB 8924 verwendet.

5. Verfahren nach einem der vorhergehenden Ansprüche, dadurch gekennzeichnet, dass man ADP in an sich bekannter Weise durch Phosphorylierung mit Phosphoenolpyruvat in Gegenwart von Pyruvatkinase unter Bildung von ATP und Pyruvat umsetzt und letzteres mit NADH in Gegenwart von Lactatdehydrogenase zu NAD oxidiert und dieses nachweist.

6. Verfahren nach einem der Ansprüche 1 bis 4, dadurch gekennzeichnet, dass man Glycerin-1-phosphat in an sich bekannter Weise mit $O_2$ in Gegenwart von Glycerin-1-phosphatoxidase zu Dihydroxyacetonphosphat und $H_2O_2$ umsetzt und letzteres mit 4-Aminoantipyrin und einem Phenol- oder Anilinderivat in Gegenwart von Peroxidase zu einem Farbstoff umsetzt und diesen bestimmt.

7. Verfahren nach einem der Ansprüche 1 bis 4, dadurch gekennzeichnet, dass man ADP in an sich bekannter Weise durch Phosphorylierung mit Phosphoenolpyruvat in Gegenwart von Pyruvatkinase zu ATP und Pyruvat umsetzt, Pyruvat mit Sauerstoff und Pyruvatoxidase zu Acetylphosphat, $CO_2$ und $H_2O_2$ reagieren lässt und letzteres mit 4-Aminoantipyrin und einem Phenol- oder Anilinderivat in Gegenwart von Peroxidase zu einem Farbstoff umsetzt, der bestimmt wird.

8. Reagens zur Bestimmung von Triglyceriden, enthaltend Lipase, ATP, Glycerokinase und ein System zur Bestimmung von Glycerin-1-phosphat oder ADP und gegebenenfalls Esterase, gekennzeichnet durch einen Gehalt an Glycerokinase aus Bacillus stearothermophilus in Kombination mit einem Aktivator aus der Gruppe der Detergentien oder/und Phenolderivate oder/und Anilinderivate.

9. Reagens nach Anspruch 8, gekennzeichnet durch einen Gehalt an einem Polyäthylenoxidester, Polyäthylenoxidäther, einer Gallensäure oder/und Alkylsulfat als Detergens.

10. Reagens nach Anspruch 8 oder 9, gekennzeichnet durch einen Gehalt an einem Phenol oder Anilin, welches mit einer Carboxylgruppe, Sulfonsäuregruppe, einem, zwei oder drei Halogenatomen oder/und einer oder zwei Nitrogruppen substituiert ist.

11. Reagens nach Anspruch 9, dadurch gekennzeichnet, dass es Natriumdodecylphosphat als Aktivator und als System zur Bestimmung von ADP Phosphoenolpyruvat, Pyruvatkinase, NADH und Lactatdehydrogenase enthält.

12. Reagens nach Anspruch 11, dadurch gekennzeichnet, dass es

60 bis 200 U Lipase,
0,4 bis 10 U Esterase,
0,5 bis 10 U Glycerokinase aus Bacillus stearothermophilus,
0,5 bis 10 U Pyruvatkinase,
2 bis 10 U Lactatdehydrogenase,
0,25 bis 0,7 µMol Phosphoenolpyruvat,
0,25 bis 0,7 µMol ATP,
0,15 bis 0,3 µMol NADH,
0,3 bis 0,5 µMol Natriumdodecylsulfat,
4 bis 100 µMol $MgSO_4$ und
20 bis 100 Phosphatpuffer, pH 6,8 bis 7,5,

bezogen auf 1 ml Reagenslösung, enthält.

13. Reagens nach Anspruch 8 bis 10, dadurch gekennzeichnet, dass es als Aktivator Dichlorphenol, Natriumcholat und einen Alkylarylpolyäthylenoxidäther und als System zur Bestimmung von ADP Phosphoenolpyruvat, Pyruvatkinase, NADH und Lactatdehydrogenase enthält.

14. Reagens nach Anspruch 13, dadurch gekennzeichnet, dass es

0,6 bis 10 U Esterase,
60 bis 300 U Lipase,
0,5 bis 10 U Glycerokinase aus Bacillus stearothermophilus,
0,5 bis 10 U Pyruvatkinase,
2 bis 10 U Lactatdehydrogenase,
0,15 bis 3 µMol NADH,
0,25 bis 0,7 µMol Phosphoenolpyruvat,
0,25 bis 0,7 µMol ATP,
1,5 bis 4 µMol 2,3-Dichlorphenol,
1,5 bis 5,0 mg Natriumcholat,
0,8 bis 2 µl Octylphenylpolyäthylenoxidester,
150 bis 300 µMol $MgSO_4$ und
40 bis 100 µMol Tris/Weinsäurepuffer, pH 7,0 bis 7,6 enthält, bezogen auf 1 ml Reagenslösung.

15. Reagens nach Anspruch 8 bis 10, dadurch gekennzeichnet, dass es als Aktivator 2,4-Dichlorphenol, Isotridecylpolyäthylenoxidäther und Natriumcholat und als System zur Bestimmung von Glycerin-1-phosphat Glycerinphosphatoxidase, Peroxidase und 4-Aminoantipyrin enthält.

16. Reagens nach Anspruch 15, dadurch gekennzeichnet, dass es
0,6 bis 10 U Esterase,
60 bis 300 U Lipase,
0,5 bis 10 U Glycerokinase aus Bacillus stearothermophilus,
2 bis 10 U Glycerinphosphatoxidase,
0,3 bis 1 µMol ATP,
3 bis 10 µMol 2,4-Dichlorphenol,
1,5 bis 5,0 mg Natriumcholat,
1,5 bis 10 µl Isotridecylpolyäthylenoxidäther,
150 bis 300 µMol Magnesiumsulfat,
40 bis 100 µMol Puffer, pH 7,5 bis 8,5,
bezogen auf 1 ml Reagenslösung, enthält.

17. Reagens nach einem der Ansprüche 8 bis 16, dadurch gekenzeichnet, dass es in einer festen Trägersubstanz imprägniert ist.

**Patent Claims**

1. Process for the determination of triglycerides by ester splitting by means of lipase and optionally of esterase with the formation of fatty acids and glycerol, phosphorylation of the glycerol with ATP in the presence of glycerokinase with the formation of glycerol-1-phosphate and ADP and determination of one of these two substances, characterised in that there is used a glycerokinase from Bacillus stearothermophilis in combination with an activator of the group consisting of detergents and/or phenol derivatives and/or aniline derivatives.

2. Process according to claim 1, characterised in that as activator one uses a detergent of the group consisting of polyethylene oxide esters or ethers, the bile acids or alkyl sulphates.

3. Process according to claim 1 or 2, characterised in that as activator one uses a phenol or aniline which is substituted with a carboxyl group, sulphonic acid group, one, two or three halogen atoms and/or one or two nitro groups.

4. Process according to one of the preceding claims, characterised in that one uses a Bacillus stearothermophilis NCIB 8924.

5. Process according to one of the preceding claims, characterised in that one reacts ADP in known manner by phosphorylation with phosphoenol pyruvate in the presence of pyruvate kinase with the formation of ATP and pyruvate and oxidises the latter with NADH in the presence of lactate dehydrogenase to give NAD and determines this.

6. Process according to one of claims 1 to 4, characterised in that one reacts glycerol-1-phosphate in known manner with $O_2$ in the presence of glycerol-1-phosphate oxidase to give dihydroxyacetone phosphate and $H_2O_2$ and reacts the latter with 4-aminoantipyrine and a phenol or aniline derivative in the presence of peroxidase to give a coloured material and determines this.

7. Process according to one of claims 1 to 4, characterised in that one reacts ADP in known manner by phosphorylation with phosphoenol pyruvate in the presence of pyruvate kinase to give ATP and pyruvate, allows pyruvate to react with oxygen and pyruvate oxidase to give acetyl phosphate, $CO_2$ and $H_2O_2$ and reacts the latter with 4-aminoantipyrine and a phenol or aniline derivative in the presence of peroxidase to give a coloured material, which is determined.

8. Reagent for the determination of triglycerides, containing lipase, ATP, glycerokinase and a system for the determination of glycerol-1-phosphate or ADP and optionally esterase, characterised by a content of glycerokinase from Bacillus stearothermophilis in combination with an activator of the group consisting of detergents and/or phenol derivatives and/or aniline derivatives.

9. Reagent according to claim 8, characterised by a content of a polyethylene oxide ester, polyethylene oxide ether, or a bile acid and/or alkyl sulphate as detergent.

10. Reagent according to claim 8 or 9, characterised by a content of a phenol or aniline which is substituted by a carboxyl group, sulphonic acid group, one, two or three halogen atoms and/or one or two nitro groups.

11. Reagent according to claim 9, characterised in that it contains sodium dodecyl phosphate as activator and, as the system for the determination of ADP, phosphoenol pyruvate, pyruvate kinase, NADH and lactate dehydrogenase.

12. Reagent according to claim 11, characterised in that it contains
60 to 200 U lipase,
0.4 to 10 U esterase,
0.5 to 10 U glyxcerokinase from Bacillus stearothermophilis,
0.5 to 10 U pyruvate kinase,
2 to 10 U lactate dehydrogenase,
0.25 to 0.7 µmol phosphoenol pyruvate,
0.25 to 0.7 µmol ATP,
0.15 to 0.3 µmol NADH,
0.3 to 0.5 µmol sodium dodecyl sulphate,
4 to 100 µmol $MgSO_4$ and
20 to 100 µmol phosphate buffer, pH 6.8 to 7.5, referred to 1 ml of reagent solution.

13. Reagent according to claims 8 to 10, characterised in that, as activator, it contains dichlorophenol, sodium cholate and an alkyl aryl polyethylene oxide ether and, as system for the determination of ATP, phosphoenol pyruvate, pyruvate kinase, NADH and lactate dehydrogenase.

14. Reagent according to claim 13, characterised in that it contains
0.6 to 10 U esterase,
60 to 300 U lipase,
0.5 to 10 U glycerokinase from Bacillus stearothermophilis,
0.5 to 10 U pyruvate kinase,
2 to 10 U lactate dehydrogenase,
0.15 to 3 µmol NADH,
0.25 to 0.7 µmol phosphoenol pyruvate,
0.25 to 0.7 µmol ATP,
1.5 to 4 µmol 2,3-dichlorophenol,
1.5 to 5.0 mg sodium cholate,
0.8 to 2 µl octylphenylpolyethylene oxide ester,
150 to 300 µmol $MgSO_4$ and
40 to 100 µmol tris/tartaric acid buffer, pH 7.0 to 7.6, referred to 1 ml of reagent solution.

15. Reagent according to claims 8 to 10, characterised in that, as activator, it contains 2,4-dichlorophenol, isotridecylpolyethylene oxide ether and sodium cholate and, as system for the determination of glycerol 1-phosphate, glycerol phosphate oxidase, peroxidase and 4-aminoantipyrine.

16. Reagent according to claim 15, characterised in that it contains
0.6 to 10 U esterase,
60 to 300 U lipase,
0.5 to 10 U glycerokinase from Bacillus stearothermophilis,
2 to 10 U glycerol phosphate oxidase,
0.3 to 1 µmol ATP,
3 to 10 µmol 2,4-dichlorophenol,
1.5 to 5.0 mg sodium cholate,

1.5 to 10 µl isotridecylpolyethylene oxide ether,
150 to 300 µmol magnesium sulphate,
40 to 100 µmol buffer, pH 7.5 8.5, referred to 1 ml
of reagent solution.

17. Reagent according to one of claims 8 to 16, characterised in that it is impregnated into a solid carrier substance.

## Revendications

1. Procédé de détermination de triglycérides par coupure d'ester au moyen de lipase et le cas échéant d'estérase avec formation d'acides gras et de glycérol, phosphorylation de glycérol par l'ATP en présence de glycérolkinase avec formation de glycérol-1-phosphate et d'ADP et détermination d'une de ces deux substances formées, caractérisé en ce qu'on utilise une glycérokinase de Bacillus stearothermophilus en combinaison avec un activateur du groupe des détergents et/ou des dérivés du phénol et/ou des dérivés de l'aniline.

2. Procédé suivant la revendication 1, caractérisé en ce qu'on utilise comme activateur un détergent du groupe des esters ou éthers de polyoxyéthylène, des acides biliaires ou des alcoylsulfates.

3. Procédé suivant la revendication 1 ou 2, caractérisé en ce qu'on utilise comme activateur un phénol ou une aniline qui est substitué par un groupe carboxyle, un groupe acide sulfonique, un, deux ou trois atomes d'halogène et/ou un ou deux groupes nitro.

4. Procédé suivant l'une des revendications précédentes, caractérisé en ce qu'on utilise un Bacillus stearothermophilus NCIB 8924.

5. Procédé suivant l'une des revendications précédentes, caractérisé en ce qu'on fait réagir l'ADP d'une manière connue en soi, par phosphorylation avec du pyruvate de phosphoénol en présence de pyruvatekinase avec formation d'ATP et de pyruvate et en ce qu'on oxyde ce dernier avec du NADH en présence de lactatedéshydrogénase, en NAD et en ce qu'on met en évidence celui-ci.

6. Procédé suivant l'une des revendications 1 à 4, caractérisé en ce qu'on fait réagir du glycérol-1-phosphate d'une manière connue en soi avec $O_2$ en présence de glycérol-1-phosphatoxydase en dihydroxyacétonephosphate et $H_2O_2$, et en ce qu'on fait réagir ce dernier avec la 4-aminoantipyrine et un dérivé du phénol ou de l'aniline en présence de peroxydase pour donner un colorant et déterminer celui-ci.

7. Procédé suivant l'une des revendications 1 à 4, caractérisé en ce qu'on fait réagir l'ADP d'une manière connue en soi par phosphorylation avec du pyruvate de phosphoénol en présence de pyruvatekinase pour donner de l'ATP et du pyruvate, en ce qu'on fait réagir le pyruvate avec l'oxygène et la pyruvate-oxydase pour donner du phosphate d'acétyle, $CO_2$ et $H_2O_2$, et en ce qu'on fait réagir ce dernier avec la 4-aminoantipyrine et un dérivé du phénol ou de l'aniline en présence de peroxydase pour donner un colorant que l'on détermine.

8. Réactif pour la détermination des triglycérides, contenant de la lipase, de l'ATP, de la glycérokinase et un système pour la détermination du glycérol-1-phosphate ou de l'ADP et le cas échéant de l'estérase, caractérisé en ce qu'il contient de la glycérokinase de Bacillus stearothermophilus en combinaison avec un activateur du groupe des détergents et/ou des dérivés du phénol ou/et des dérivés de l'aniline.

9. Réactif suivant la revendication 8, caractérisé en ce qu'il contient un ester de polyoxyéthylène, un éther de polyoxyéthylène, un acide biliaire ou/et un alcoylsulfate comme détergent.

10. Réactif suivant la revendication 8 ou 9, caractérisé en ce qu'il contient un phénol ou une aniline qui sont substitués par un groupe carboxyle, un groupe acide sulfonique, un, deux ou trois atomes d'halogène ou/et un ou deux groupes nitro.

11. Réactif suivant la revendication 9, caractérisé en ce qu'il contient du dodécylphosphate de sodium comme activateur et comme système de détermination de l'ADP du pyruvate de phosphoénol, de la pyruvatekinase, du NADH et de la lactatedéshydrogénase.

12. Réactif suivant la revendication 11, caractérisé en ce qu'il contient
60 à 200 U de lipase,
0,4 à 10 U d'estérase,
0,5 à 10 U de glycérokinase de Bacillus stearothermophilus,
0,5 à 10 U de pyruvatekinase,
2 à 10 U de lactatedeshydrogénase,
0,25 à 0,7 µmole de pyruvate de phosphoénol
0,25 à 0,7 µmole d'ATP
0,15 à 0,3 µmole de NADH,
0,3 à 0,5 µmole de dodécylsulfate de sodium,
4 à 100 µmoles de $MgSO_4$ et
20 à 100 µmoles de tampon au phosphate, pH 6,8 à 7,5 rapportés à 1 ml de solution de réactif.

13. Réactif suivant l'une des revendictions 8 à 10, caractérisé en ce qu'il contient comme activateur du dichlorophénol, du cholate de sodium et un éther d'alcoylarylpolyoxyéthylène et comme système pour la détermination de l'ADP du pyruvate de phosphoénol, de la pyruvatekinase, du NADH et de la lactatedéshydrogénase.

14. Réactif suivant la revendication 13, caractérisé en ce qu'il contient
0,6 à 10 U d'estérase,
60 à 300 U de lipase,
0,5 à 10 U de glycérokinase de Bacillus stearothermophilus,
0,5 à 10 U de pyruvatekinase,
2 à 10 U de lactatedéhydrogénase,
0,15 à 3 µmole de NADH,
0,25 à 0,7 µmole de pyruvate de phosphoénol,
0,25 à 0,7 µmole d'ATP,
1,5 à 4 µmole de 2,3-dichlorophénol,
1,5 à 5,0 mg de cholate de sodium,
0,8 à 2 µl d'ester d'octylphénylpolyoxyéthylène
150 à 300 µmoles de $MgSO_4$ et
40 à 100 µmoles de tampon tris/acide tartrique, pH 7,0 à 7,6, rapportés à 1 ml de solution de réactif.

15. Réactif suivant l'une des revendications 8 à 10, caractérisé en ce qu'il contient comme activateur du 2,4-dichlorophénol, de l'éther d'isotridécylpolyoxyéthylène et du cholate de sodium et comme système pour la détermination du glycérol-1-phosphate de la glycérolphosphatoxydase, de la péroxydase et de la 4-amino-antipyrine.

16. Réactif suivant la revendication 15, caractérisé en ce qu'il contient
0,6 à 10 U d'estérase,
60 à 300 U de lipase,
0,5 à 10 U de glycérokinase de Bacillus stearothermophilus,
2 à 10 U de glycérolphosphatoxydase,
0,3 à 1 μmole d'ATP,
3 à 10 s de 2,4-dichlorophénol,
1,5 à 5,0 mg de cholate de sodium,
1,5 à 10 μl d'éther d'isotridécylpolyoxyéthylène,
150 à 300 μmoles de sulfate de magnésium,
40 à 100 μmoles de tampon, pH 7,5 à 8,5 rapportés à 1 ml de solution de réactif.

17. Réactif suivant l'une des revendications 8 à 16, caractérisé en ce qu'il est imprégné dans une substance support solide.